# FASCICULE DE BREVET EUROPEEN

(11) **EP 0 809 473 B1**
(45) Date de publication et mention de la délivrance du brevet: **21.10.1998**
(21) Numéro de dépôt: 96901395.2
(22) Date de dépôt: 15.01.1996
(51) Int. Cl.: A61B 17/72, A61F 2/02, A61F 2/30

(54) **DISPOSITIF D'ALLONGEMENT PERFECTIONNE D'OS LONGS**
VORRICHTUNG ZUM VERLÄNGERN VON LÄNGLICHEN KNOCHEN
IMPROVED LONG BONE ELONGATION DEVICE

(30) Priorité: 13.02.1995 FR 9501628
(43) Date de publication de la demande: 03.12.1997
(73) Titulaire: MEDINOV-AMP, 42300 Roanne (FR)
(72) Inventeur: COTY, Alain, F-95450 Sagy (FR); L'HOMME, Michel, F-92500 Rueil-Malmaison (FR); PALEY, Dror, Baltimore, MD 21208 (US); FAVREUL, Emmanuel, F-67300 Strasbourg (FR); PERETTI, Giovani, I-20122 Milan (IT)
(74) Mandataire: Obolensky, Michel
(86) Numéro de dépôt international: FR9600065
(87) Numéro de publication internationale: WO9625117

(56) Documents cités:
- DE-U- 8 907 561
- FR-A- 2 267 080
- US-A- 4 173 796
- US-A- 5 156 605
- PROCEEDINGS OF THE ANNUAL INTERNATIONAL CONFERENCE OF THE IEEE ENGINEERING IN MEDECINE AND BIOLOGY SOCIETY, 28 - 31 Octobre 1993, SAN DIEGO USA, pages 909-910, XP000436947 K.KOSHIJI: "externally-coupled transcutaneous energy transmission system for a totally implantable artificial heart"

## Description

La présente invention concerne les dispositifs d'allongement d'os longs tels que le fémur, tibias, humérus et se rapporte plus particulièrement à un dispositif de commande progressive d'un tel dispositif d'allongement.

Le traitement des inégalités de longueur fémorale ou des cas de nanisme est actuellement effectué par des dispositifs d'allongement d'os purement mécaniques.

Bien que satisfaisants quant à la fonction demandée, ces dispositifs appelés fixateurs externes sont contraignants pour le patient et ont un taux de complications très élevé.

On connaît également des dispositfs d'allongement d'os longs qui comprennent un clou centro-médullaire qui comporte un fourreau tubulaire, une allonge pouvant coulisser axialement à l'intérieur du fourreau, le fourreau et l'allonge étant munis d'organes de solidarisation avec deux parties de l'os séparées par une ostéotomie et des moyens pour déplacer l'allonge dans le fourreau, aptes à conférer au clou centro-médullaire ainsi qu'à l'os dans lequel il est fixé, un allongement progressif obtenu automatiquement ou par une action externe, ledit système, étant entièrement inclus dans l'organisme et l'os concerné.

Un tel dispositif d'allongement comportant les caractéristiques du préambule de la revendication 1 est connu du brevet américain US-A-5 156 605. Un autre dispositif similaire comprenant un moteur electrique intégré et alimenté par un champ magnétique extérieur est également connu de la demande FR-A-2 267 080.

Un autre clou de ce type décrit dans la demande de brevet européen n° 0 346 247 déposée le 22 mai 1989 par la Demanderesse, est conformé à partir de plusieurs éléments aptes à lui conférer un allongement progressif résultant d'une rotation partielle appliquée sur le membre concerné et le retour à la position d'origine.

On connaît de plus un dispositif d'allongement d'os long comprenant un clou centro-médullaire qui comporte un fourreau tubulaire, une allonge pouvant coulisser axialement à l'intérieur du fourreau, le fourreau et l'allonge étant munis d'organes de solidarisation avec deux parties de l'os fracturé et des moyens pour déplacer l'allonge dans le fourreau, ces moyens de déplacement comprenant un fil d'alliage à mémoire de forme disposé axialement dans le fourreau entre une extrémité de ce dernier et une extrémité de l'allonge à laquelle la partie terminale contigue du fil est fixée, des moyens mécaniques pour transformer la force de compression produite par le fil à mémoire de forme sous l'effet de cycles successifs de chauffage et de refroidissement dudit fil, en une force axiale de distraction exercée sur l'allonge et tendant à la faire sortir partiellement du fourreau, des moyens de chauffage par induction du fil à mémoire de forme et un générateur d'énergie de chauffage par induction pourvu d'une antenne émettrice destinée à entourer le membre d'un patient dont l'os est muni du clou centro-médullaire et adapté pour actionner cycliquement les moyens de chauffage du fil à mémoire de forme afin de provoquer des avances progressives de l'allonge pour entraîner un allongement du clou et donc un allongement de l'os.

Un tel clou centro-médullaire décrit dans la demande de brevet européen n°0 346 247 permet d'obtenir un allongement pour un os long tel que le fémur pouvant aller jusqu'à 10 cm et il évite les problèmes septiques entraînés par l'utilisation d'un dispositif d'allongement à commande externe ainsi que les risques de déviation angulaire au cours de l'allongement grâce au guidage centro-médullaire.

Ce clou d'allongement permet également la formation d'un cal osseux suivie d'une consolidation d'os lamellaire de qualité sensiblement supérieure à celle obtenue par les fixateurs externes, et ne nécessite théoriquement que deux temps opératoires, l'un pour la pose et l'autre pour le retrait.

De plus, il limite le nombre de cicatrices et surtout il limite leur dimension, ce qui représente un atout psychologique important pour le patient.

Enfin, ce type de fixateur interne conserve au patient une activité sans handicap pendant la deuxième moitié de la durée du traitement et supprime les problèmes d'esthétique.

En revanche, le cal (ou osteon) formé entre les deux moitiés de l'os et qui assure leur liaison est nécessairement brisé par les rotations répétées de 15° (30 fois par jour) de la jambe pour obtenir 2 mm d'allongement quotidien, ce qui tend à provoquer de vives douleurs chez le patient. De plus, le chou manque de force linéaire d'allongement, en raison de ses très faibles dimensions, ce qui limite l'allongement maximum possible.

Le dispositif pour l'allongement d'os longs mettant en oeuvre un fil d'alliage à mémoire de forme décrit dans la demande de brevet français n° 94 13 244 publication: FR-A-2 726 460 déposée le 4 novembre 1994 au nom de la Demanderesse, ne nécessite plus de faire appel à des rotations répétées de la jambe de sorte que les inconvénients provoqués par les ruptures successives du cal sont supprimés.

Cependant, ce dernier dispositif présente comme celui décrit dans la demande de brevet européen n° 0 346 247, l'inconvénient de ne pas permettre de connaître à tout moment la valeur de l'action mécanique exercée sur le chou centro-médullaire et de ce fait sur les deux parties du membre à allonger, ce qui rend très difficile un contrôle précis de l'opération d'allongement qui s'exerce généralement sur une période de l'ordre de trois à dix mois.

L'invention vise à remédier à cet inconvénient en créant un système d'allongement médullaire qui permette d'une part d'assurer des courses d'allongement très précises et d'autre part, d'effectuer un contrôle permanent de l'allongement.

L'invention a donc pour objet un dispositif d'allongement d'os longs, comprenant un clou centro-médullaire qui comporte un fourreau tubulaire, une allonge pouvant coulisser axialement à l'intérieur du fourreau, le fourreau et l'allonge comprenant des moyens permettant leur solidarisation avec deux parties de l'os séparées par une ostéotomie et des moyens pour déplacer l'allonge par rapport au fourreau aptes à conférer au clou centro-médullaire ainsi qu'à l'os destiné, à le recevoir, un allongement progressif, les moyens pour déplacer l'allonge par rapport au fourreau comprenant disposé dans le fourreau, un moteur électrique associé à un réducteur de vitesse d'entraînement d'un ensemble vis-écrou de déplacement de l'allonge par rapport au fourreau, ce déplacement étant quantifié par un capteur de position entrainé par le réducteur, et des moyens d'alimentation du moteur électrique et de commande automatique de la valeur et du sens du déplacement de l'allonge dans le fourreau par l'ensemble vis-écrou actionné par le moteur électrique, lesdits moyens d'alimentation et de commande comportant un transformateur comprenant une première partie destinée à être implantée sous la peau du patient et une seconde partie destinée à être appliquée sur la peau du patient en regard de la première partie caractérisé en ce que la première partie du tranformateur comporte un circuit magnétique portant sur deux branches d'extrémité des enroulements secondaires d'alimentation du moteur électrique dans deux sens contraires et sur une branche intermédiaire, un enroulement primaire de réception de signaux de position de la vis de l'ensemble vis-écrou provenant d'un capteur de position entraîné par le réducteur, tandis que la seconde partie du transformateur destinée à être disposée en regard de la première partie par application contre la peau du patient comporte un circuit magnétique portant sur deux branches d'extrémité des enroulements primaires connectés à un circuit d'alimentation destinés à coopérer avec les enrouleurs secondaires de la première partie et sur une branche intermédiaire, un enroulement secondaire destiné à coopérer avec l'enroulement primaire de la première partie pour transmettre les signaux de position de l'ensemble vis-écrou à un circuit de filtrage et de mise en forme.

L'invention sera mieux comprise à la lecture de la description qui va suivre, donnée uniquement à titre d'exemple et faite en se référant aux dessins annexés, sur lesquels :
- la Fig.1 est une vue en coupe partielle d'un dispositif d'allongement progressif intramédullaire motorisé selon l'invention;
- la Fig.2 est un schéma synoptique du circuit électronique de commande qui fait partie du dispositif de la figure 1;
- la Fig.3 montre un schéma électronique du circuit d'alimentation du primaire du transformateur qui fait partie du dispositif de commande de la figure 2;
- la Fig.4 est un schéma synoptique d'un circuit de filtrage des signaux de sortie indiquant l'état de l'allongement du système;
- la Fig.5 est un schéma électrique du circuit de commande d'alimentation du moteur électrique du dispositif d'allongement suivant l'invention;
- la Fig.6 est un schéma électrique représentant le circuit de transmission des impulsions de comptage, des pas d'allongement jusqu'à l'enroulement primaire du transformateur de transmission de données qui fait partie du dispositif selon l'invention; et
- la Fig.7 est un schéma synoptique des moyens de comptage et d'affichage des signaux relatifs à l'état d'allongement du dispositif d'allongement intramédullaire suivant l'invention.

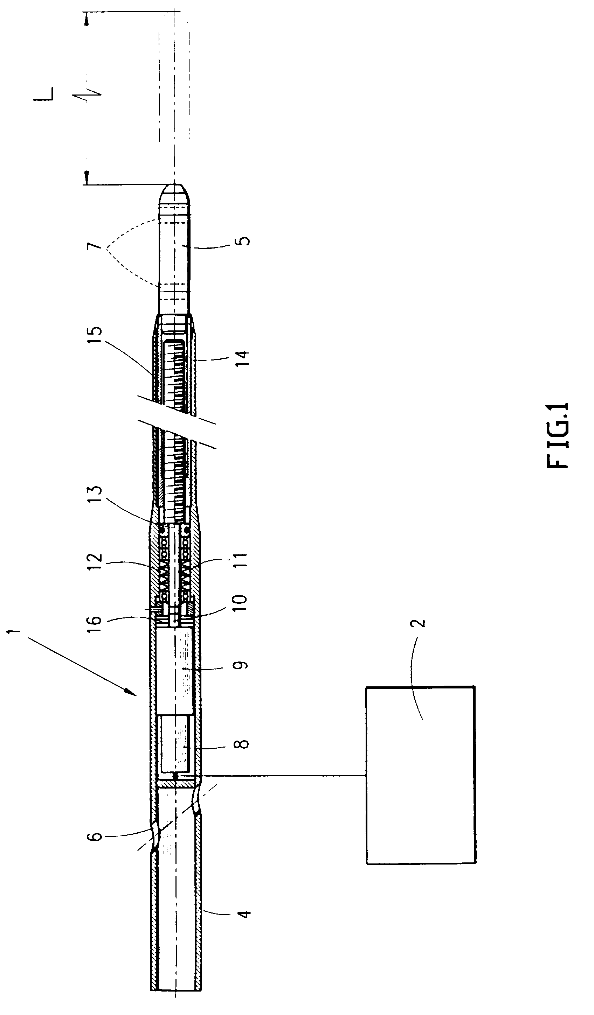

Le dispositif d'allongemennt intramédullaire suivant l'invention comporte, comme représenté schématiquement à la figure 1, un clou centro-médullaire 1 destiné à être placé dans le canal médullaire d'un os long tel qu'un fémur dont l'allongement doit être assuré et un circuit électronique 2 de commande de ce dispositif.

Le clou centro-médullaire 1 comprend un fourreau tubulaire 4 et une allonge 5 pouvant coulisser axialement à l'intérieur du fourreau, le fourreau et l'allonge étant munis de trous transversaux respectifs 6 et 7 de réception de vis ou goupilles non représentées destinées à être fixées dans des parties correspondantes de l'os à allonger afin d'immobiliser par rapport à ces parties de l'os, d'une part, le fourreau 4 et d'autre part, l'allonge 5 par fixation à l'os de la partie de cette allonge extérieure au fourreau 4.

Dans le fourreau 4 est disposé un moteur électrique d'entraînement 8 alimenté en énergie électrique et commandé par le circuit électronique 2 d'une manière qui sera décrite par la suite.

Au moteur électrique 8 est accouplé un réducteur de vitesse 9 dont l'arbre de sortie 10 est relié par l'intermédiaire d'un dispositif amortisseur axial 11 réalisé à l'aide d'un empilage de rondelles élastiques 12, à une tige 13 comportant une portion filetée 14 coopérant avec une jupe 15 formant écrou qui prolonge l'allonge 5 à l'intérieur du fourreau 4.

Le moteur électrique 8 est avantageusement réalisé d'un diamètre de 7,5 mm tandis que le réducteur de vitesse présente également un diamètre très faible de l'ordre de 8 mm par exemple, et son rapport de réduction est par exemple de 8200.

La tige 13 à portion filetée 14 entraîne simultanément un capteur incrémental 16 qui dans le présent exemple, délivre trois impulsions par tour.

Les informations issues du capteur incrémental après avoir été retransmises au travers de la peau d'un membre équipé du dispositif suivant l'invention, permettent de quantifier le déplacement par rapport au fourreau de l'allonge dont la course totale L est représentée à la Fig 1.

On voit que l'ensemble constitué par le moteur 8 et le réducteur 9 est extrêmement miniaturisé, ce qui permet sa mise en place dans le dispositif d'allongement intramédullaire suivant l'invention.

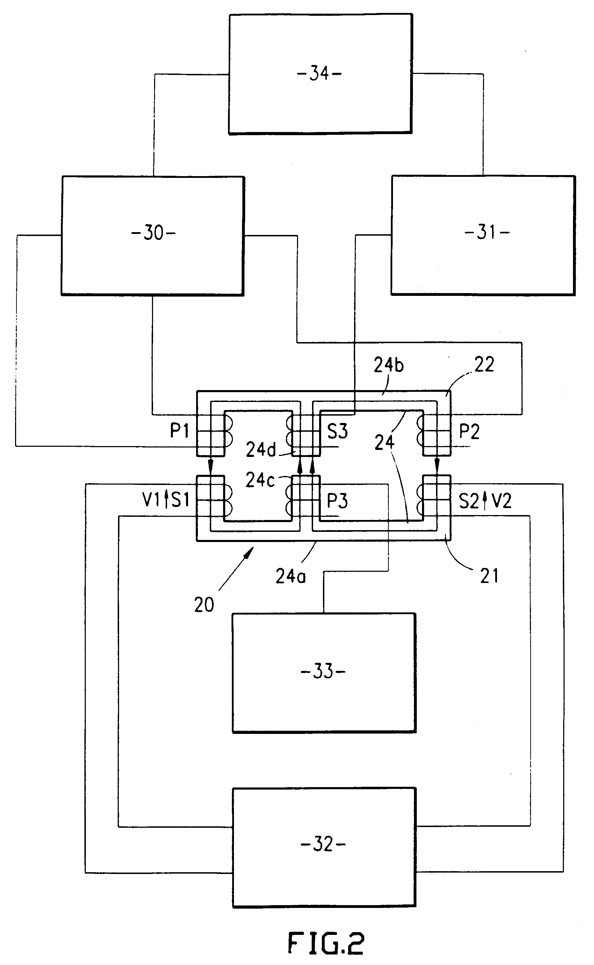

En se référant à la figure 2, on voit que le circuit de commande électrique du dispositif suivant l'invention comporte principalement un transformateur 20 auquel sont connectés divers circuits d'une manière qui sera décrite par la suite.

L'introduction d'une pile dans l'organisme posant des problèmes de sécurité, il est apparu préférable de mettre en oeuvre un dispositif qui permette de transmettre l'énergie électrique au travers de la peau d'un patient.

Les puissances mises en jeu étant faibles et les notions de rendement sans grande signification, la Demanderesse a montré qu'il est possible d'utiliser un transformateur à grand entrefer.

Le transformateur 20 est donc physiquement scindé en deux parties.

Il comporte une première partie 21 implantée sous la peau du patient et une seconde partie 22 destinée à être appliquée sur la peau du patient en regard de la première partie 21 dès que l'on souhaite provoquer un déplacement du système d'allongement.

Les fonctions à réaliser par le dispositif selon l'invention sont multiples, car il faut non seulement transmettre de l'énergie de l'extérieur vers l'intérieur du corps du patient, mais aussi pouvoir recevoir des informations sur la valeur du déplacement de l'allonge 5 du clou centro-médullaire par rapport à son fourreau 4.

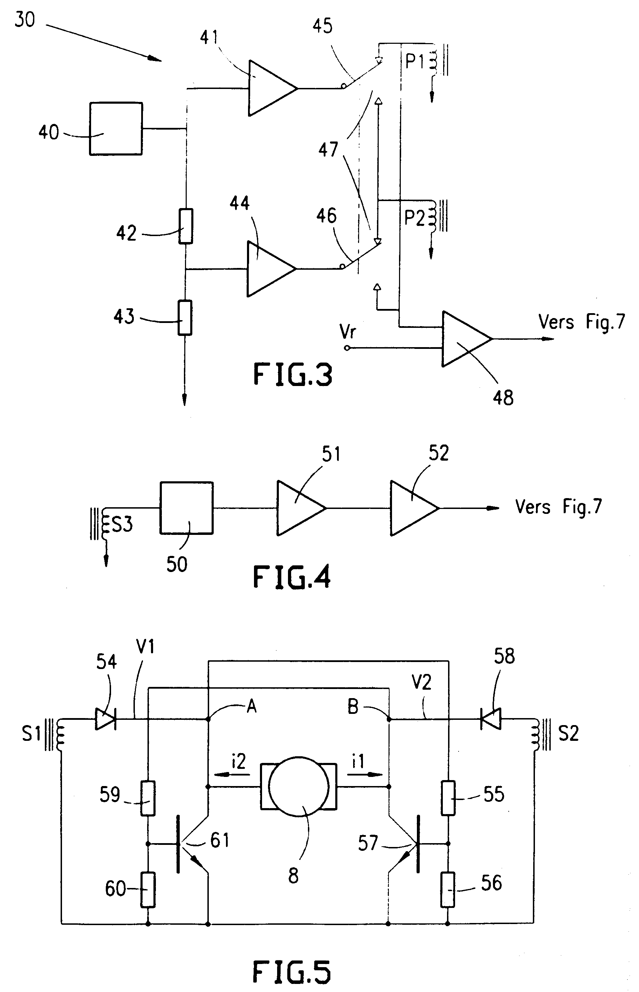

Ces fonctions sont obtenues en associant aux première et seconde parties 21 et 22 du transformateur 20 des circuits électroniques appropriés qui vont être décrits en détail en référence aux figures 3 à 7.

Le transformateur 20 représenté à la figure 2 comporte comme tout transformateur, un circuit magnétique 24 sur lequel sont disposés des enroulements.

Des enroulements primaires P1, P2 sont montés sur des branches d'extrémité du circuit magnétique 24a de la seconde partie 22 du transformateur et sont destinés à coopérer avec les enroulements secondaires correspondants S1, S2 montés sur des branches d'extrémité du circuit magnétique 24b de la première partie 21 du transformateur pour transmettre l'énergie électrique de l'extérieur de la peau vers le moteur électrique 8.

Moyennant des dispositifs électroniques qui vont être décrits par la suite, lorsque l'enroulement primaire P1 est alimenté, une tension V1 est induite dans l'enroulement secondaire S1 et le moteur 8 incorporé au clou centro-médullaire tourne dans un sens déterminé.

A l'inverse, lorsque l'enroulement primaire P2 est alimenté, une tension V2 apparaît aux bornes de l'enroulement secondaire S2 et le moteur 8 tourne dans le sens contraire.

Le transformateur représenté à la figure 2 comporte en outre un enroulement primaire P3 monté sur une branche intermédiaire 24c du circuit magnétique 24a de la première partie 21 et coopérant avec un enroulement secondaire S3 monté sur une branche intermédiaire 24d du circuit magnétique 24b de la seconde partie 22 du transformateur.

L'enroulement P3 reçoit des trains d'impulsions chaque fois que le capteur de position 16 effectue un tiers de tour et ces impulsions sont captées par le secondaire S3 afin d'être mises en forme avant comptage d'une manière qui sera décrite par la suite.

On voit également sur la figure 2 que le circuit magnétique 24 formé des première et seconde parties 24a et 24b est asymétrique en ce sens que les branches intermédiaires 24c et 24d de ses deux parties 24a, 24b sont disposées à des distances inégales des deux branches d'extrémité correspondantes de manière à lever toute indétermination quant au sens de rotation du moteur.

Cette disposition crée un détrompage de la position des poles de la première partie 21 par rapport à ceux de la seconde partie 22 du transformateur 20.

La fonction principale de la seconde partie 22 du transformateur consiste à délivrer de l'énergie à la première partie 21. Or, en regardant la figure 2, si l'on observe le trajet emprunté par le flux principalement dans une branche, on s'aperçoit qu'une partie de celui-ci est dérivée dans l'autre branche.

La tension V1 induite dans le secondaire S1 par le flux principal tend à faire tourner le moteur 8 dans un sens.

La tension V2 induite dans le secondaire S2 tend au contraire à faire tourner le moteur 8 dans l'autre sens et donc à le freiner.

Afin d'éviter ces inconvénients, il suffit d'alimenter l'enroulement P2 avec une valeur de courant juste nécessaire pour créer un flux de compensation qui annule la tension V2.

Un raisonnement identique peut être effectué lorsque c'est l'enroulement P2 qui doit être alimenté. Il faut alors compenser simultanément le flux dérivé en faisant circuler un courant de compensation de valeur appropriée dans l'enroulement primaire P1.

A cet effet, le circuit de la figure 2 comporte en outre un circuit électronique 30 d'alimentation des enroulements primaires P1 et P2 de la seconde partie 22 du transformateur 20.

Il est en outre prévu un circuit 31 de filtrage des signaux de sortie du secondaire S3 de ladite seconde partie. Les enroulements secondaires S1 et S2 de la première partie 21 sont connectés aux bornes d'un circuit 32 de détermination du sens de rotation du moteur 8 du clou centro-médullaire, tandis que le primaire P3 de la premièr partie 21 du transformateur est connecté à la sortie d'un circuit 33 générateur de signaux de pas correspondant aux signaux délivrés par le capteur de position 16 du dispositif de la figure 1.

Enfin, les circuits 30 et 31 sont connectés à un circuit 34 de comptage et d'affichage des signaux relatifs à l'état d'allongement du dispositif d'allongement intramédullaire selon l'invention.

Le circuit 30 d'alimentation des primaires P1 et P2 du transformateur représenté en détail à la figure 3 comporte principalement un oscillateur 40 ayant une fréquence de l'ordre de 10000 Hz, dont la sortie est connectée directement à l'entrée d'un premier amplificateur 41 et par l'intermédiaire d'un diviseur de tension formé de deux résistances 42,43, à l'entrée d'un second amplificateur 44.

Ainsi, l'amplificateur 41 est l'amplificateur de puissance principal alors que l'amplificateur 44 est un amplificateur de compensation.

Les sorties des amplificateurs 41 et 44 sont connectées à des contacts mobiles respectifs couplés 45, 46 d'un inverseur 47 à deux voies. Un contact fixe associé au contact mobile 45 est relié à une première borne de l'enroulement primaire P1 tandis que son autre contact fixe est relié à une première borne de l'enroulement primaire P2. L'un des contacts fixes associé au contact mobile 46 est connecté lui aussi à la première borne de l'enroulement P2 alors que son autre contact fixe est relié à la première borne de l'enroulement P1.

La première borne de l'enroulement primaire P1 est en outre connectée à une entrée d'un amplificateur différentiel 48 dont l'autre entrée est connectée à une tension de référence Vr. Les autres bornes des enroulements primaires P1 et P2 sont connectées à la masse comme est également connectée à la masse la résistance 43 du diviseur de tension 42,43.

L'oscillateur 40 alimente soit directement l'amplificateur 41 de puissance principal, soit l'amplificateur de compensation 44 par l'intermédiaire du pont diviseur 42,43. Le rôle de ce diviseur de tension est d'ajuster précisément le flux de compensation.

Pour choisir le sens de rotation du moteur 8, il suffit d'actionner le commutateur 47, de manière à diriger le courant principal vers l'un ou l'autre des deux enroulements primaires P1 ou P2, l'enroulement primaire qui est connecté à l'amplificateur de puissance principal 41 déterminant le sens de rotation du moteur, tandis que l'enroulement primaire connecté à l'amplificateur de compensation 44 reçoit un courant de compensation de sens et d'intensité appropriés pour compenser le flux dérivé qui le parcourt du fait de l'alimentation de l'autre enroulement primaire par l'amplificateur de puissance principal.

Ainsi qu'on l'a expliqué plus haut, grâce à un capteur incrémental tel que le capteur 16 entraîné par l'arbre de sortie du réducteur 9 du dispositif d'allongement centro-médullaire représenté à la figure 1, il est possible de compter le nombre de tours effectué par la tige filetée 13,14 et connaître ainsi la valeur du déplacement en translation de l'allonge 5 par rapport au fourreau 4.

Cependant, pour procéder à une telle évaluation, il est nécessaire de connaître le sens dans lequel s'effectue la rotation de la tige filetée 13,14. Cette information est obtenue grâce à la présence du comparateur 48 dont la fonction consiste à déterminer celui des enroulements primaires P1 ou P2 qui reçoit le courant d'excitation principal provenant de l'oscillateur 40 et à délivrer un signal logique correspondant.

A cet effet, le comparateur 48 délivre un signal de sortie à deux états O ou 1 après comparaison du signal qu'il reçoit sur son entrée reliée soit à l'enroulement primaire P1, soit à l'enroulement primaire P2 selon la position des contacts mobiles 45,46 de l'inverseur 47, avec la tension de référence Vr appliquée à son autre entrée.

Pour que les impulsions de comptage soient aisément discriminées, il est nécessaire que la fréquence de leur porteuse soit très différente de celle utilisée pour transférer l'énergie.

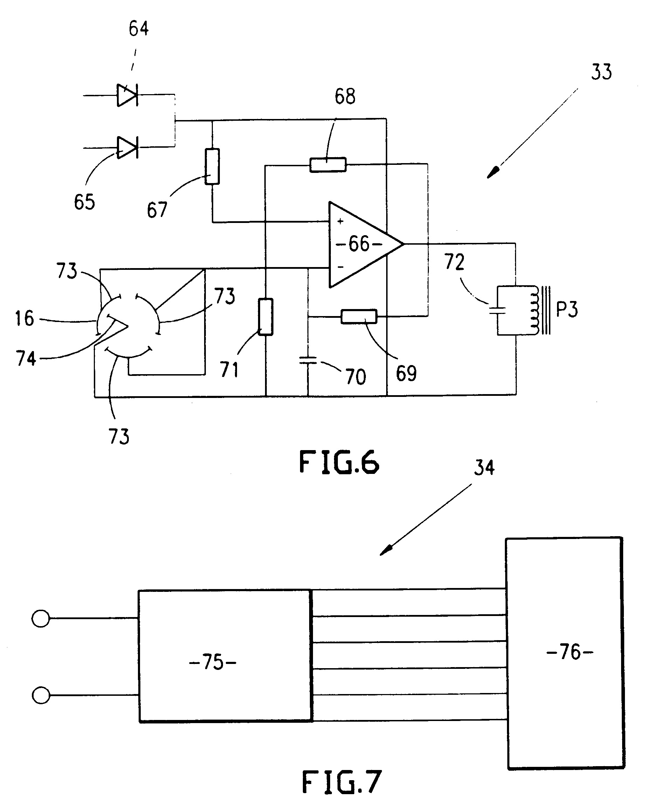

La fréquence de l'oscillateur d'alimentation 40 ayant été choisie égale à 10 KHz, la fréquence de la porteuse des signaux de comptage est avantageusement choisie égale à 170 kHz. La porteuse des signaux de comptage est engendrée par le circuit 33 qui sera décrit en détail en référence à la figure 6 et appliquée à l'enroulement primaire P3 de la première partie 21 du transformateur 20.

Les signaux reçus par l'enroulement primaire P3 sont transmis à l'enroulement secondaire S3 de la seconde partie 22 du transformateur extérieur au corps du patient et appliqués au circuit 31 qui va maintenant être décrit en référence à la figure 4.

Ce circuit comporte connecté à l'enroulement secondaire S3, un filtre 50 de la tension induite aux bornes d'un enroulement S3 qui extrait l'information souhaitée et dont la sortie est connectée à son tour par l'intermédiaire d'un détecteur 51 à un circuit de mise en forme 52 destiné à convertir des signaux détectés en signaux carrés et connecté comme représenté à la figure 2 à une entrée de comptage du circuit 34 qui sera décrit en référence à la figure 7.

Le seul fait que le transformateur représenté à la figure 2 comporte deux enroulements secondaires séparés S1 et S2 qui peuvent devenir à la demande générateurs d'une tension ne suffit pas pour alimenter correctement le moteur 8 dans l'un ou l'autre des deux sens de rotation.

Il faut de plus que le circuit redresseur associé à chacun de ces enroulements secondaires débite uniquement dans le moteur 8 et soit donc commandé par la tension d'alimentation elle-même.

Pour répondre à cette condition, la Demanderesse a conçu un circuit électronique qui constitue une sorte de pont redresseur auto-commandé. Un tel agencement désigné par la référence générale 32 sur la figure 2 va être décrit en détail en référence à la figure 5.

On voit sur cette figure que l'enroulement secondaire S1 du transformateur 20 est connecté en série avec une diode 54 et deux résistances 55, 56 formant diviseur de tension. Au point de jonction des résistances 55 et 56 est connectée la base d'un transistor 57 dont le trajet émetteur-collecteur est relié à une borne du moteur électrique 8. La diode 54 est en outre connectée à l'autre borne du moteur électrique 8.

L'enroulement secondaire S2 est de même connecté en série avec une diode 58 et deux résistances 59, 60 formant diviseur de tension. Au point de jonction des résistances 59 et 60 est connectée la base d'un autre transistor 61 dont le trajet émetteur-collecteur est relié à ladite autre borne du moteur 8. La diode 58 est également connectée à la borne du moteur électrique 8 reliée au trajet émetteur-collecteur du transistor 57.

Par ailleurs, les résistances 56 et 60 et les émetteurs des transistors 57 et 61 sont connectés en parallèle aux bornes des enroulements secondaires S1 et S2 opposés à celles reliées aux diodes 54 et 58.

Le fonctionnement de l'agencement décrit en référence à la figure 5 est le suivant.

On suppose tout d'abord que seul l'enroulement secondaire S1 est alimenté et par conséquent générateur de courant. Si S1 est générateur, une tension V1 redressée apparaît à la sortie de la diode 54 et grâce au pont diviseur constitué par les résistances 55 et 56, le transistor 57 est convenablement polarisé et devient donc conducteur. Un courant il peut ainsi circuler dans le moteur 8 et provoquer sa rotation dans un sens.

De même, lorsque seul l'enroulement secondaire S2 est générateur, c'est le transistor 61 qui est rendu conducteur en raison de la présence sur le diviseur de tension constitué par les résistances 59 et 60, de la tension V2 de sortie de la diode 58.

Alors, un courant i2 de sens inverse du courant il traverse le moteur 8 et provoque sa rotation en sens contraire.

On voit donc que l'on obtient ainsi un circuit qui se comporte comme un pont redresseur dans lequel le sens du courant est commandé par les tensions qui apparaissent aux bornes de l'un ou l'autre des deux enroulements secondaires S1 et S2.

Au cours de la description du circuit d'alimentation des enroulements primaires P1 et P2 représenté à la figure 3, il a été indiqué que celui des enroulements primaires P1 et P2 qui ne doit pas participer à l'alimentation du moteur 8 pour l'entraîner dans un sens déterminé, reçoit de l'oscillateur 40 un courant d'alimentation de compensation du flux dérivé qui le traverse et qui est engendré dans le circuit magnétique 24b du transformateur 20 par celui des enroulements primaires P2 ou P1 alimenté.

La première partie 21 du transformateur 20 du dispositif suivant l'invention a également pour fonction de transmettre des impulsions de comptage au travers de l'enroulement primaire P3.

La figure 6 représente le circuit qui permet d'assurer cette fonction.

Ce circuit qui porte sur la figure 2 la référence générale 33, comporte principalement deux diodes 64,65 connectées respectivement aux points A et B du circuit de la figure 5 sur lesquels apparaissent les tensions redressées de sortie des enroulements secondaires S1 et S2 lorsqu'ils sont alimentés. Les cathodes des diodes 64,65 sont connectées ensemble à une entrée positive d'un amplificateur opérationnel 66 par l'intermédiaire d'une résistance 67 et d'autre part à une borne d'alimentation de l'amplificateur opérationnel 66. La borne négative de l'amplificateur opérationnel 66 est connectée à la sortie du capteur de position 16.

Par ailleurs, une résistance 68 est connectée entre l'entrée positive de l'amplificateur 66 et sa sortie, une autre résistance 69 est connectée entre l'entrée négative de l'amplificateur 66 et sa sortie, tandis qu'un condensateur 70 est connecté entre l'entrée négative de l'amplificateur opérationnel 66 et la masse.

Une résistance 71 est connectée entre l'entrée positive de l'amplificateur 66 et la masse.

L'agencement ainsi constitué forme un multivibrateur dont la sortie débite sur l'enroulement primaire P3.

Enfin, la sortie de l'amplificateur opérationnel 66 est connectée à l'enroulement primaire P3 auquel est connecté en paralléle un condensateur 72.

Le circuit accordé constitué par l'enroulement primaire P3 et le condensateur 72 est accordé sur une fréquence égale à 170 kHz qui est la fréquence du multivibrateur.

L'amplificateur opérationnel 66 autour duquel est constitué le multivibrateur est toujours alimenté à la même polarité quel que soit le sens de rotation du moteur 8 grâce aux diodes 64 et 65.

L'entrée du multivibrateur 66 est connectée aux plots fixes 73 du capteur incrémental 16 alors que son curseur 74 est relié au zéro électrique.

Ainsi, le multivibrateur normalement bloqué, délivre un train d'impulsions chaque fois que le curseur 74 se trouve entre deux plots fixes 73, c'est à dire trois fois par tour de la tige filetée 13,14 du dispositif d'allongement représenté à la figure 1.

Le circuit représenté à la figure 7 qui porte le numéro de référence 34 sur la figure 2, est constitué d'un compteur-décompteur 75 comprenant une première entrée reliée à la sortie du circuit de mise en forme 31 représenté à la figure 4 et dont une seconde entrée est reliée à la sortie du comparateur 48 de détermination du sens de rotation du moteur d'entraînement 8 associé à l'inverseur 47 du circuit d'alimentation de la Fig. 3.

Le compteur-décompteur est connecté à son tour à un circuit d'affichage de la valeur et du sens du déplacement de l'allonge 5 par rapport au fourreau 4 du dispositif d'allongement intramédullaire de l'invention.

Les trois fonctions principales dudit dispositif suivant l'invention que sont la transmission d'énergie électrique au travers de la peau du patient, la commande du sens de rotation du moteur d'entraînement du dispositif d'allongement et la connaissance du déplacement obtenu, peuvent être réalisées en utilisant un seul et unique transformateur.

Les dispositifs électroniques associés à ce transformateur permettent à la fois d'utiliser au mieux l'énergie transmise et de retransmettre les informations sans que ces diverses fonctions interfèrent entre elles.

On voit donc que grâce à l'agencement qui vient d'être décrit, on réalise les opérations d'allongement d'un os long tel qu'un fémur de manière parfaitement contrôlée et que l'on peut en particulier intervenir sur les pas de l'allongement à obtenir.

Par ailleurs, le dispositif suivant l'invention permet une lecture permanente des valeurs des allongements obtenus.

Enfin, le dispositif suivant l'invention comporte une partie située entièrement à l'intérieur de l'organisme du patient et pouvant être commandé de l'extérieur par simple contact avec la peau, ce qui évite toute traversée des chairs et de la peau et par conséquent supprime les problèmes septiques.

## Revendications

1. Dispositif d'allongement d'os longs, comprenant un clou centro-médullaire qui comporte un fourreau tubulaire (4), une allonge (5) pouvant coulisser axialement à l'intérieur du fourreau (4), le fourreau et l'allonge comprenant des moyens (6;7) permettant leur solidarisation avec deux parties de l'os séparées par une ostéotomie et des moyens pour déplacer l'allonge (5) par rapport au fourreau (4) aptes à conférer au clou centro-médullaire ainsi qu'à l'os destiné à le recevoir, un allongement progressif, les moyens pour déplacer l'allonge (5) par rapport au fourreau (4) comprenant disposé dans le fourreau (4) un moteur électrique (8) associé à un réducteur de vitesse (9) d'entraînement d'un ensemble (13,14, 15) vis-écrou de déplacement de l'allonge (5) par rapport au fourreau, ce déplacement étant quantifié par un capteur de position (16) entrainé par le réducteur (9), et des moyens (2) d'alimentation du moteur électrique (8) et de commande automatique de la valeur et du sens du déplacement de l'allonge (5) dans le fourreau (4) par l'ensemble vis-écrou (13,14,15) actionné par le moteur électrique (8), lesdits moyens d'alimentation et de commande comportant un transformateur (20) comprenant une première partie (21) destinée à être implantée sous la peau du patient et une seconde partie (22) destinée à être appliquée sur la peau du patient en regard de la première partie (21), caractérisé en ce que la première partie (21) du tranformateur comporte un circuit magnétique (24a) portant sur deux branches d'extrémité des enroulements secondaires (S1, S2) d'alimentation du moteur électrique (8) dans deux sens contraires et sur une branche intermédiaire (24c), un enroulement primaire (P3) de réception de signaux de position de la vis (13,14) de l'ensemble vis-écrou (13,14,15) provenant du capteur de position (16) entraîné par le réducteur (9), tandis que la seconde partie (22) du transformateur (20) destinée à être disposée en regard de la première partie par application contre la peau du patient comporte un circuit magnétique (24b) portant sur deux branches d'extrémité des enroulements primaires (P1, P2) connectés à un circuit d'alimentation (30) destinés à coopérer avec les enrouleurs secondaires (S1, S2) de la première partie (21) et sur une branche intermédiaire (24d), un enroulement secondaire (S3) destiné à coopérer avec l'enroulement primaire (P3) de la première partie (21) pour transmettre les signaux de position de l'ensemble vis-écrou (13,14,15) à un circuit (31) de filtrage et de mise en forme.

2. Dispositif d'allongement suivant la revendication 1, caractérisé en ce que l'ensemble vis-écrou du clou centro-médullaire comporte une tige (13) comportant une portion filetée (14) qui coopère avec une jupe (15) formant écrou qui prolonge l'allonge (5) à l'intérieur du fourreau (4), la tige (13) à portion filetée (14) étant reliée à l'arbre de sortie (10) du réducteur de vitesse (9).

3. Dispositif d'allongement suivant la revendication 2, caractérisé en ce que la tige (13) à portion filetée (14) est reliée à l'arbre de sortie (10) du réducteur (9) par l'intermédiaire d'un amortisseur axial (11).

4. Dispositif d'allongement suivant l'une des revendications 2 et 3, caractérisé en ce que la tige (13) à partie filetée (14) entraîne le capteur de position (16) constitué par un capteur incrémental de la position de ladite tige (13) et par conséquent du déplacement de l'allonge (5) par rapport au fourreau (4).

5. Dispositif d'allongement suivant l'une des revendications 1 et 4, caractérisé en ce que les branches intermédiaires (24c, 24d) des circuits magnétiques (24a, 24b) des première et seconde parties (21,22) du transformateur sont disposées à des distances inégales des branches d'extrémités correspondantes de chacun desdits circuits magnétiques, ce qui assure un détrompage de la position des pôles de la première partie (21) par rapport à ceux de la seconde partie (22) du transformateur (20).

6. Dispositif d'allongement suivant l'une des revendications 1 à 5, caractérisé en ce que le circuit d'alimentation (30) des enroulements primaires (P1, P2) de la seconde partie (22) du transformateur comporte un oscillateur (40) engendrant des signaux d'alimentation d'une première fréquence, ledit oscillateur étant connecté directement à un amplificateur de puissance principal (41) et par l'intermédiaire d'un diviseur (42,43), à un amplificateur de compensation (44), l'amplificateur de puissance principal (41) et l'amplificateur de compensation (44) étant connectés aux enroulements primaires (P1, P2) par l'intermédiaire d'un inverseur (47) destiné à assurer l'application du signal de sortie de l'amplificateur de puissance (41) à l'un des enroulements primaires (P1, P2) devant être alimenté et l'application du signal de sortie de l'amplificateur de compensation à l'autre enroulement primaire (P2, P1) dans lequel l'effet du flux dérivé résultant de l'alimentation dudit enroulement primaire alimenté doit être compensé.

7. Dispositif d'allongement suivant l'une des revendications 1 à 6, caractérisé en ce que l'enroulement secondaire (S3) porté par le circuit magnétique de la seconde partie (22) du transformateur est connecté à un circuit (50) de filtrage des signaux de position du système vis-écrou (13,14,15), lui-même connecté par l'intermédiaire d'un détecteur (51) à un circuit (52) de mise en forme.

8. Dispositif d'allongement suivant l'une des revendications 1 à 6, caractérisé en ce qu'il comporte en outre un circuit (32) de détermination du sens de rotation du moteur électrique (8) d'entrainement du système vis-écrou (13,14,15), ce circuit comprenant deux circuits redresseurs (55,58,60,61, 59,54,56,57) connectés respectivement aux enroulements secondaires (S1, S2) et dont la conduction provoquée par l'alimentation de l'un de ces enroulements secondaires provoque la rotation du moteur dans un sens ou dans l'autre.

9. Dispositif d'allongement suivant l'une des revendications 1 à 8, caractérisé en ce que l'enroulement primaire (P3) de la première partie (21) du transformateur (20) est relié à un circuit (33) générateur de signaux de position angulaire de l'ensemble vis-écrou (13,14,15) qui comporte le capteur angulaire (16) de la position d'une tige filetée (13,14) entraînée par le réducteur (9) et dont la sortie est connectée à l'entrée négative d'un amplificateur opérationnel (66) dont l'entrée positive est connectée par l'intermédiaire de diodes (64,65) de même polarité à des points (A, B) du circuit (30) de détermination du sens de rotation du moteur électrique (8) sur lesquels apparaît la tension redressée de sortie de chacun des enroulements secondaires (S1, S2), l'amplificateur opérationnel faisant partie d'un multivibrateur (66,68,69, 70,71) qui est connecté à un circuit accordé formé de l'enroulement primaire (P3) et d'un condensateur (72) accordé sur une fréquence différente de celle de l'oscillateur (40) du circuit d'alimentation.

10. Dispositif d'allongement suivant l'une des revendications 1 à 8, caractérisé en ce qu'il comporte en outre un circuit (34) de comptage et d'affichage de la valeur et du sens du déplacement de l'allonge (5) par rapport au fourreau (4) comprenant un compteur-décompteur (75) dont une première entrée est connectée à la sortie du circuit (31) de filtrage et de mise en forme des signaux de position de l'ensemble vis-écrou reçus par l'enroulement secondaire (S3) de ladite seconde partie (22) du transformateur, une seconde entrée du compteur-décompteur étant connectée à la sortie d'un comparateur (48) associé à l'inverseur (47) du circuit (30) d'alimentation des enroulements primaires (P1, P2) de la seconde partie (22) du transformateur et destiné à délivrer un signal à deux états correspondant respectivement à l'alimentation par l'amplificateur de puissance principal (41) de l'un ou de l'autre des enroulements primaires (P1,P2) et un circuit d'affichage (76) relié à la sortie dudit compteur-décompteur.

## Claims

1. Device for extending long bones, comprising an intramedullary pin which has a tubular sleeve (4), an extension piece (5) capable of sliding axially within the sleeve (4), the sleeve and the extension piece comprising means (6;7) enabling them to be attached to two parts of the bone separated by an osteotomy and means for moving the extension piece (5) relative to the sleeve (4), adapted to cause progressive elongation of the intramedullary pin and of the bone intended to receive it, the means for moving the extension piece (5) relative to the sleeve (4) comprising an electric motor (8), arranged in the sleeve (4), associated with a speed reducer (9) driving a nut-and-bolt assembly (13, 14, 15) for moving the extension piece (5) relative to the sleeve, this movement being quantified by a position sensor (16) driven by the reducer (9), and means (2) for supplying the electric motor (8) and automatically controlling the magnitude and direction of movement of the extension piece (5) in the sleeve (4) by the nut-and-bolt assembly (13, 14, 15) actuated by the electric motor (8), said supply and control means comprising a transformer (20) having a first part (21) adapted to be implanted under the patient's skin and a second part (22) adapted to be applied to the patient's skin opposite the first part (21), characterised in that the first part (21) of the transformer comprises a magnetic circuit (24a) bearing secondary windings (S1, S2) on two end branches for supplying the electric motor (8) in two opposite directions and a primary winding (P3) on an intermediate branch (24c) for receiving signals indicating the position of the bolt (13, 14) of the nut-and-bolt assembly (13, 14, 15) coming from the position sensor (16) driven by the reducer (9), while the second part (22) of the transformer (20) intended to be arranged opposite the first part by being applied to the patient's skin comprises a magnetic circuit (24b) carrying primary windings (P1, P2), on two end branches, connected to a supply circuit (30) and intended to co-operate with the secondary windings (S1, S2) of the first part (21) and, on an intermediate branch (24d), a secondary winding (S3) adapted to co-operate with the primary winding (P3) of the first part (21) to transmit the position signals of the nut-and-bolt assembly (13, 14, 15) to a filtering and shaping circuit (31).

2. Extension device according to claim 1, characterised in that the nut-and-bolt assembly of the intramedullary pin comprises a rod (13) having a threaded portion (14) which cooperates with a flange (15) forming a nut which prolongs the extension piece (5) inside the sleeve (4), the rod (13) with its threaded portion (14) being connected to the output shaft (10) of the speed reducer (9).

3. Extension device according to claim 2, characterised in that the rod (13) with its threaded portion (14) is connected to the output shaft (10) of the speed reducer (9) via an axial shock absorber.

4. Extension device according to one of claims 2 and 3, characterised in that the rod (13) with its threaded portion (14) drives the position sensor (16) consisting of an incremental sensor of the position of said rod (13) and hence of the movement of the extension piece (5) relative to the sleeve (4).

5. Extension device according to one of claims 1 and 4, characterised in that the intermediate branches (24c, 24d) of the magnetic circuits (24a, 24b) of the first and second parts (21, 22) of the transformer are located at unequal distances from the corresponding end branches of each of said magnetic circuits, thus correcting the position of the poles of the first part (21) relative to those of the second part (22) of the transformer (20).

6. Extension device according to one of claims 1 to 5, characterised in that the supply circuit (30) of the primary windings (P1, P2) of the second part (22) of the transformer comprises an oscillator (40) which generates supply signals at a first frequency, said oscillator being directly connected to a main power amplifier (41) and, via a divider (42, 43), to a compensating amplifier (44), the main power amplifier (41) and the compensating amplifier (44) being connected to the primary windings (P1, P2) via an inverter (47) adapted to ensure that the output signal of the power amplifier (41) is applied to the one of the primary windings (P1, P2) which is to be supplied and that the output signal from the compensating amplifier is applied to the other primary winding (P2, P1) in which the effect of the derived flux resulting from the supply of said supplied primary winding has to be compensated.

7. Extension device according to one of claims 1 to 6, characterised in that the secondary winding (S3) carried by the magnetic circuit of the second part (22) of the transformer is connected to a circuit (50) for filtering the position signals of the nut-and-bolt assembly (13, 14, 15), which is itself connected to a shaping circuit (52) via a detector (51).

8. Extension device according to one of claims 1 to 6, characterised in that it further comprises a circuit (32) for determining the direction of rotation of the electric drive motor (8) for the nut-and-bolt assembly (13, 14, 15), this circuit comprising two rectifier circuits (55, 58, 60, 61, 59, 54, 56, 57) connected to the secondary windings (S1, S2) respectively and the conduction of which, caused by supplying one of these secondary windings, causes the motor to rotate in one direction or the other.

9. Extension device according to one of claims 1 to 8, characterised in that the primary winding (P3) of the first part (21) of the transformer (20) is connected to a circuit (33) generating signals indicating the angular position of the nut-and-bolt assembly (13, 14, 15) which comprises the angular sensor (16) of the position of a threaded rod (13, 14) driven by the reducer (9) and the output of which is connected to the negative input of an operational amplifier (66) the positive input of which is connected via diodes (64, 65) of the same polarity to points (A, B) on the circuit (30) for determining the direction of rotation of the electric motor (8) at which the rectified output voltage from each of the secondary windings (S1, S2) appears, the operational amplifier forming part of a multivibrator (66, 68, 69, 70, 71) which is connected to a tuned circuit formed by the primary winding (P3) and a capacitor (72) tuned to a different frequency from that of the oscillator (40) of the supply circuit.

10. Extension device according to one of claims 1 to 8, characterised in that it further comprises a circuit (34) for counting and displaying the magnitude and direction of movement of the extension piece (5) relative to the sleeve (4) comprising a forward/reverse counter (75) a first input of which is connected to the output of the filtering and shaping circuits (31) of the signals indicating the position of the nut-and-bolt assembly received by the secondary winding (S3) of said second part (22) of the transformer, a second input of the forward/reverse counter being connected to the output of a comparator (48) associated with the inverter (47) of the supply circuit (30) for the primary windings (P1, P2) of the second part (22) of the transformer and intended to deliver a signal in two states which correspond, respectively, to the supply by the main power amplifier (41) of one or other of the primary windings (P1, P2) and a display circuit (76) connected to the output of said forward/reverse counter.

## Patentansprüche

1. Vorrichtung zum Verlängern von länglichen Knochen, bestehend aus einem Knochenmark-Nagel aus einem rohrförmigen Gehäuse (4), einem Verlängerungsstück (5), das im Innern des Gehäuses (4) axial verschiebbar sitzt, Mitteln (6;7), über die eine Befestigung des Gehäuses und des Verlängerungsstückes an zwei Teilen des Knochens möglich ist, die osteotomisch getrennt worden sind, und Mitteln, um das Verlängerungsstück (5) relativ zu dem Gehäuse (4) zu verrücken und dadurch eine fortschreitende Verlängerung des Knochenmark-Nagels und des Knochens, in dem sich der Nagel befindet, zu erhalten, wobei die Mittel zur Verrückung des Verlängerungsstücks (5) relativ zu dem Gehäuse (4), untergebracht in dem Gehäuse (4), einen Elektro-Motor (8) mit einem Geschwindigkeitsverminderer (9) zum Antrieb einer Schrauben-Mutter-Einheit (13,14,15) zwecks Verrückung des Verlängerungsstücks (5) relativ zu dem Gehäuse umfassen und die Verrückung durch einen vom dem Geschwindigkeitsverminderer (9) angetriebenen Positionsfühler (16) erfaßt wird, und aus Mitteln (2), um den Elektro-Motor zu versorgen und automatisch den Wert und die Richtung der Verrückung des Verlängerungsstücks (5) in dem Gehäuse (4) über die durch den Elektro-Motor (8) angetriebene Schrauben-Mutter-Einheit (13,14,15) zu steuern, wobei die Mittel zur Versorgung und Steuerung eine Transformator (20) umfassen, der aus einem ersten, unter der Haut des Patienten implantierten Teil (21) und einem zweiten, auf der Haut des Patienten, gegenüber dem ersten Teil (21) angeordneten Teil (22) besteht, **dadurch gekennzeichnet,** daß der erste Teil (21) des Transformators einen magnetischen Kreis (24a) aufweist, der auf zwei äußeren Armen Sekundär-Spulen (S1,S2) zur Versorgung des Elektro-Motors (8) in zwei zueinander entgegengesetzten Richtungen und auf einem mittleren Arm (24c) eine Primär-Spule (P3) zur Aufnahme von Positionssignalen der Schraube (13,14) der Schrauben-Mutter-Einheit (13,14,15) trägt, welche Signale von dem durch den Geschwindigkeitsverminderer (9) angetriebenen Positionsfühler (16) kommen, und daß der zweite Teil (22) des Transformators (20), der dem ersten Teil gegenüber auf der Haut des Patienten angeordnet ist, einen magnetischen Kreis (24b) aufweist, der auf zwei äußeren Armen Primärspulen (P1,P2), die mit einem Versorgungskreis (30) verbunden sind und mit den Sekundärspulen (S1,S2) des ersten Teils (21) zusammenarbeiten, und auf einem mittleren Arm (24d) eine Sekundärspule (S3) trägt, die mit der Primärspule (P3) des ersten Teils (21) zusammenarbeitet, um die Positionssignale der Schrauben-Mutter-Einheit (13,14,15) an einen Filter- und Formungs-Kreis zu übertragen.

2. Vorrichtung zum Verlängern nach Anspruch 1, **dadurch gekennzeichnet,** daß die Schrauben-Mutter-Einheit des Knochenmark-Nagels eine Stange (3) mit einem Gewinde (14) aufweist, das mit einem Überzugsteil (15) in Form einer Mutter zusammenwirkt, das das Verlängerungsstück (5) im Innern des Gehäuses (4) verlängert, wobei die Stange (13) mit dem Gewindeteil (14) mit der Ausgangswelle (10) des Geschwindigkeitsverminderers (9) verbunden ist.

3. Vorrichtung zum Verlängern nach Anspruch 2, **dadurch gekennzeichnet,** daß die Stange (13) mit dem Gewindeteil (14) mit der Ausgangswelle (10) des Geschwindigkeitsverminderers (9) unter Zwischenschaltung eines Axial-Puffers (11) verbunden ist.

4. Vorrichtung zum Verlängern nach einem der Ansprüche 2 und 3, **dadurch gekennzeichnet,** daß die Stange (13) mit dem Gewindeteil (14) den Positionsfühler (16) antreibt, der als Inkrement-Fühler für die Position der Stange (13) und damit für die Verrückung des Verlängerungsstücks (5) relativ zu dem Gehäuse (4) ausgebildet ist.

5. Vorrichtung zum Verlängern nach einem der Ansprüche 1 und 4, **dadurch gekennzeichnet,** daß die mittleren Arme (24c,24d) der magnetischen Kreise (24a,24b) der ersten und zweiten Teile (21,22) des Transformators ungleiche Abstände von den äußeren Armen der magnetischen Kreise haben, was ein Irren bezüglich der Position der Pole des ersten Teils (21) relativ zu den Polen des zweiten Teils (22) des Transformators kenntlich macht.

6. Vorrichtung zum Verlängern nach einem der Ansprüche 1 bis 5, **dadurch gekennzeichnet,** daß der Versorgungskreis (30) für die Primärspulen (P1,P2) des zweiten Teils (22) des Transformators einen Oszillator (40) aufweist, der Versorgungssignale mit einer ersten Frequenz erzeugt, daß der Oszillator direkt mit einem Haupt-Leistungs-Verstärker (41) und unter Zwischenschaltung eines Teilers (42,43) mit einem Kompensations-Verstärker (44) verbunden ist, daß der Haupt-Leistungs-Verstärker (41) und der Kompensations-Verstärker (44) mit den Primärspulen (P1,P2) unter Zwischenschaltung eines Umschalters (47) derart verbunden sind, daß das Ausgangssignal des Leistungsverstärkers (41) an der Primärspule (P1,P2) liegt, die versorgt werden soll, und das Ausgangssignal des Kompensationsverstärkers an der anderen Primärspule (P2,P1) liegt, in welcher auf Grund eines resultierenden Flusses eine Kompensation der Versorgung erfolgt.

7. Vorrichtung zum Verlängern nach einem der Ansprüche 1 bis 6, **dadurch gekennzeichnet,** daß die Sekundärspule (S3) des magnetischen Kreises des zweiten Teils (22) des Transformators mit einem Kreis (50) zum Filtern der Positionssignale des Schrauben-Mutter-Systems (13,14,15) verbunden ist und daß dieser Kreis (50) unter Zwischenschaltung eines Detektors (51) mit einem Kreis (52) zum Formen verbunden ist.

8. Vorrichtung zum Verlängern nach einem der Ansprüche 1 bis 6, **dadurch gekennzeichnet,** daß diese einen Kreis (32) aufweist, der die Richtung der Rotation des Elektro-Motors (8) zum Antrieb des Schrauben-Mutter-Systems (13,14,15) bestimmt, wobei dieser Kreis zwei Gleichrichter-Kreise (55,58,60,61, 59,54,56,57) aufweist, die mit den Sekundärspulen (S1,S2) verbunden sind und die durch Versorgung einer der Sekundärspulen eine Rotation des Motors in der einen oder anderen Richtung hervorrufen.

9. Vorrichtung zum Verlängern nach einem der Ansprüche 1 bis 8, **dadurch gekennzeichnet,** daß die Primärspule (P3) des ersten Teils (21) des Transformators (20) mit einem Kreis (33) verbunden ist, der Signale gemäß der Winkelposition der Schrauben-Mutter-Einheit (13,14,15) erzeugt, die den Winkel-Positionsfühler (16) zur Erfassung der Position einer Gewindestange (13,14), angetrieben durch den Verminderer (9), umfaßt, daß der Ausgang des Fühlers (16) mit dem negativen Eingang eines Operationsverstärkers (66) verbunden ist, dessen positiver Eingang unter Zwischenschaltung von Dioden (64,65) gleicher Polarität mit Punkten (A,B) des Kreises (30) zur Bestimmung des Richtungssinnes des Elektro-Motors (8) verbunden ist, an welchen Punkten die gleichgerichtete Ausgangsspannung jeder Sekundärspule (S1,S2) anliegt, und daß der Operations-Verstärker Teil eines Multivibrators (66,68,69,70.71) ist, der mit einem Kreis verbunden ist, der aus der Primärspule (P3) und einem Kondensator (72) besteht und auf eine Frequenz abgestimmt ist, die zu der Frequenz des Oszillators (40) des Versorgungskreises verschieden ist.

10. Vorrichtung zum Verlängern nach einem der Ansprüche 1 bis 8, **dadurch gekennzeichnet,** daß diese einen weiteren Kreis (34) zur Erfassung und Anzeige des Wertes und der Richtung der Verrückung des Verlängerungsstücks (5) relativ zu dem Gehäuse (4) aufweist, bestehend aus einem Aufwärts-Abwärts-Zähler (75), dessen erster Eingang mit dem Ausgang des Kreises (31) zum Filtern und Formen der Positionssignale der Schrauben-Mutter-Einheit, erhalten durch die Sekundärspule (S3) des zweiten Teils (22) des Transformators, verbunden ist, wobei ein zweiter Eingang des Aufwärts-Abwärts-Zählers mit dem Ausgang eines Komparators (48) verbunden ist, der dem Umschalter (47) des Kreises (30) zur Versorgung der Primärspulen (P1,P2) des zweiten Teils (22) des Transformators zugeordnet ist und bestimmt ist, ein Signal aus zwei Zuständen entsprechend der Versorgung der einen oder anderen der Primärspulen (P1,P2) durch den Haupt-Leistungsverstärker (41) zu liefern, und aus einem Anzeige-Kreis (76), der mit dem Ausgang des Aufwärts-Abwärts-Zählers verbunden ist.
